# EUROPEAN PATENT APPLICATION

(11) **EP 2 113 864 A2**
(43) Date of publication of application: **04.11.2009**
(21) Application number: 09251209.4
(22) Date of filing: 28.04.2009
(51) Int. Cl.: G06K 7/00, G06K 7/08

(54) **RFID to prevent reprocessing**

(30) Priority: 29.04.2008 US 111715
(71) Applicant: Ethicon Endo-Surgery, Inc., Cincinnati, OH 45242 (US)
(72) Inventor: Green, Jack, Mason Ohio 45040 (US); Leuenberger, Mark S., Loveland Ohio 45140 (US)
(74) Representative: Small, Gary James

(57) **Abstract**

An RFID identification system includes an RFID tag associated with a medical device, an RFID antenna for receiving signals issued by the RFID tag, an RFID reader identifying an RFID labeled medical device, and a computer system in communication with the RFID reader.

## Description

### BACKGROUND OF THE INVENTION

### 1. Field of the Invention

The present invention relates to a monitoring system for medical devices. In particular, the present invention relates to various systems and methodologies for monitoring medical devices through the utilization of RFID technology.

### 2. Description of the Related Art

Engineers specializing in the design and manufacture of medical instruments commonly attempt to improve previously existing medical instruments by enhancing the usage of these instruments. By improving the medical instrument, the possibility for user error is often drastically reduced. Through improved engineering, these engineers attempt to eliminate the gap between the best surgeon and the worst surgeon through careful product design. Similarly, they try to transform patient care through inventive product design. Many of their medical instruments are designed for minimally invasive procedures, resulting in quicker surgeries, lower risk of complications, less pain, shorter recovery time and lower costs.

The development of improved manufacturing techniques, advanced materials and concerns regarding contamination have led to the development of medical instruments designed for single use applications. For example, many laparoscopic devices such as, surgical staplers and trocars, are designed as single use items that are intended to be immediately disposed of after use.

A recent trend in the medical community is reprocessing of single use medical instruments, by parties other than the original equipment manufacturer, instead of discarding them after use. During reprocessing, the medical instruments are disassembled, cleaned and sterilized. They are then reassembled for future use.

However, many of the medical instruments reprocessed for further use are specifically designed only for use during a single procedure. Consequently, the performance of the medical instruments decline after reprocessing, since the components making up the medical instrument are not adapted for multiple uses and will degrade in performance when used beyond their intended life span. For example, reprocessing of the cutting devices on trocars extends these devices beyond their intended mission life and may result in duller blades. A greater force, therefore, is needed to make an initial incision, causing more trauma to the patient. In addition, the use of greater force increases the potential for error during the surgical procedure.

As reprocessing of medical instruments proliferates, it has become very difficult to identify if an item has in fact been reprocessed or if it is the original medical instrument delivered by the original manufacturer. Doctors usually do not even know if a medical instrument has been reprocessed, since the medical instrument is commonly unpacked prior to use by the doctor and any reprocessing notification is located on the packaging of the medical instrument. The FDA requires labeling of only the package of the reprocessed item with the new manufacturer information; the medical instrument itself is not required to be marked. In fact, some hospitals instruct the staff responsible for opening medical instruments and setting up the medical instruments for surgery not to inform the doctor as to whether the medical instruments have been reprocessed.

With the foregoing in mind, great problems are encountered in the utilization and proliferation of reprocessed instruments. In addition to the problems associated with reprocessed instruments, it is often desirable for hospitals and other medical facilities to monitor the equipment utilized during a medical procedure. This monitoring may allow them to determine whether unsterilized equipment is brought into a sterile zone, whether equipment is lost during a procedure, as well as identifying if the medical device about to be used is subject to a recent recall notice and enabling the protections of medical devices remaining in the sterile field at the conclusion of a procedure to ensure that stray medical devices are not left behind when closing up the patient as well as ensuring actual counts entering the sterile field have in fact left.

In addition, it is sometimes desirable to identify the specific location of medical instruments in the event they are needed or must be withdrawn from a location immediately.

With the foregoing in mind, techniques are needed for the monitoring of medical equipment utilized within hospitals and medical facilities. The present invention provides such a system.

### SUMMARY OF THE INVENTION

It is, therefore, an object of the present invention to provide an RFID identification system including an RFID tag associated with a medical device, an RFID antenna for receiving signals issued by the RFID tag, an RFID reader identifying an RFID labeled medical device, and a computer system in communication with the RFID reader.

It is also an object of the present invention to provide an RFID identification system wherein the RFID antenna is incorporated into a perimeter of an anticipated operating site.

It is another object of the present invention to provide an RFID identification system wherein the RFID antenna is incorporated into a patient drape.

It is a further object of the present invention to provide an RFID identification system wherein the RFID reader is hardwired to the computer system.

It is also an object of the present invention to provide an RFID identification system wherein the RFID reader is wirelessly connected to the computer system.

It is another object of the present invention to provide an RFID identification system wherein the RFID tag is provided with unique information about the medical device such that an RFID reader is able to pick up the medical device as it enters or exits the area under the monitoring of the RFID reader.

It is a further object of the present invention to provide an RFID identification system wherein the RFID reader is mounted within a surgical light.

It is also an object of the present invention to provide an RFID identification system wherein the RFID reader is mounted within a disposable light handle cover.

It is another object of the present invention to provide an RFID identification system wherein the RFID reader is incorporated within the ceiling of the operating room.

It is a further object of the present invention to provide an RFID identification system wherein the RFID reader is incorporated within the perimeter of the surgical table.

It is also an object of the present invention to provide an RFID identification system wherein the RFID reader is incorporated within the mayo stand where medical devices are placed upon removal from packaging.

It is another object of the present invention to provide an RFID identification system wherein the RFID reader is incorporated with within or onto the floor area around the surgical field.

It is a further object of the present invention to provide an RFID identification system wherein the RFID tag applied to a medical device includes a unique serialized identifier.

It is also an object of the present invention to provide an RFID identification system wherein the RFID reader is included with the waste container.

It is another object of the present invention to provide an RFID identification system wherein the RFID tag is attached to the inside or outside of a single use medical device for the purpose of detecting the point in time when it was first used.

It is a further object of the present invention to provide an RFID identification system wherein the medical devices include an actuation mechanism which interrupts or alters circuitry of the RFID tag such that the time/date of activation of the medical device is recorded by the circuitry within the RFID tag.

It is also an object of the present invention to provide an RFID identification system wherein sensors are attached to the RFID tags to also record specific conditions of use or misuse.

It is another object of the present invention to provide an RFID identification system wherein the RFID tag is used on or in a disposable medical device designed for limited use in conjunction with electronic capital equipment.

It is a further object of the present invention to provide an RFID identification system wherein the RFID tag is incorporated into a hospital case cart.

Other objects and advantages of the present invention will become apparent from the following detailed description when viewed in conjunction with the accompanying drawings, which set forth certain embodiments of the invention.

### BRIEF DESCRIPTION OF THE DRAWINGS

Figure 1 is a view of an operating room implementing an RFID identification system in accordance with a first embodiment of the present invention.
Figure 2 is a detailed view of the surgical table shown in Figure 1.
Figure 3 is a schematic showing operation of the RFID identification system shown in Figure 1 wherein a wired system is used to connect the RFID reader to the hospital computer system.
Figure 4 is a schematic showing operation of the RFID identification system shown in Figure 1 wherein a wireless system is used to connect the RFID reader to the hospital computer system.
Figures 5 and 6 respectively show an operating room view and a schematic in accordance with an alternate embodiment of the present invention.
Figures 7 and 8 respectively show an operating room view and a schematic in accordance with an alternate embodiment of the present invention.
Figures 9 and 10 respectively show an operating room view and a schematic in accordance with an alternate embodiment of the present invention.
Figures 11 and 12 respectively show an operating room view and a schematic in accordance with an alternate embodiment of the present invention.
Figure 13 is a schematic in accordance with an alternate embodiment of the present invention.
Figures 14 and 15 respectively show a waste container and a schematic in accordance with an alternate embodiment of the present invention.
Figures 16 and 17 respectively show a waste container and a schematic in accordance with an alternate embodiment of the present invention.
Figures 18 and 19 are respectively a trocar and a surgical stapler employing an RFID identification system in accordance with the present invention.

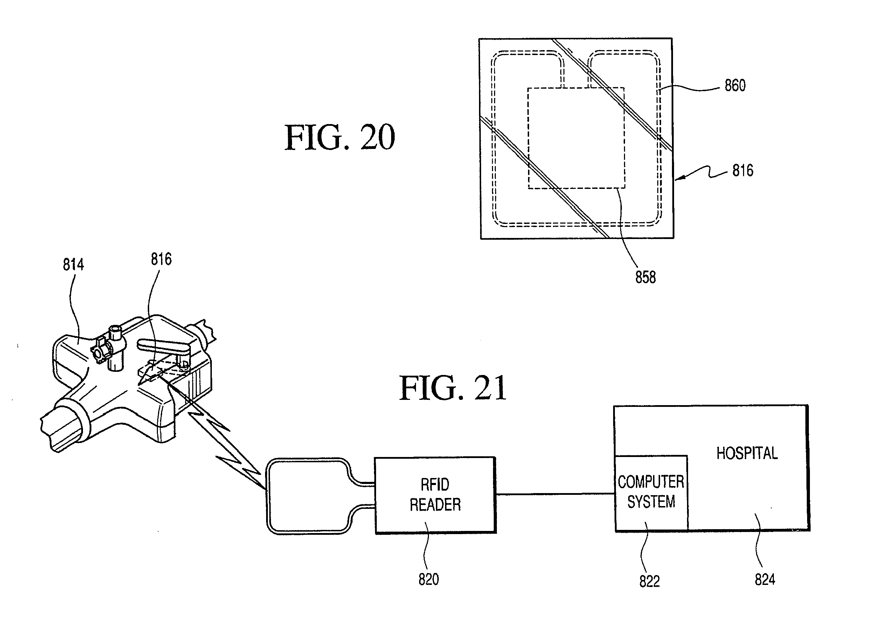

Figure 20 is a RFID tag used in the embodiments disclosed with reference to Figures 18 and 19.
Figure 21 is a schematic showing transmission of RFID signals in accordance with the embodiments presented with reference to Figures 18 and 19.
Figures 22 and 23 are respectively a surgical stapler employing an RFID identification system in accordance with the present invention and a schematic showing transmission of RFID signals.
Figures 24 and 25 respectively show an operating room view and a schematic in accordance with an alternate embodiment of the present invention.
Figures 26 and 27 respectively show a hospital case cart and a schematic in accordance with an alternate embodiment of the present invention.

### DESCRIPTION OF THE PREFERRED EMBODIMENTS

The detailed embodiment of the present invention is disclosed herein. It should be understood, however, that the disclosed embodiment is merely exemplary of the invention, which may be embodied in various forms. Therefore, the details disclosed herein are not to be interpreted as limiting, but merely as a basis for teaching one skilled in the art how to make and/or use the invention.

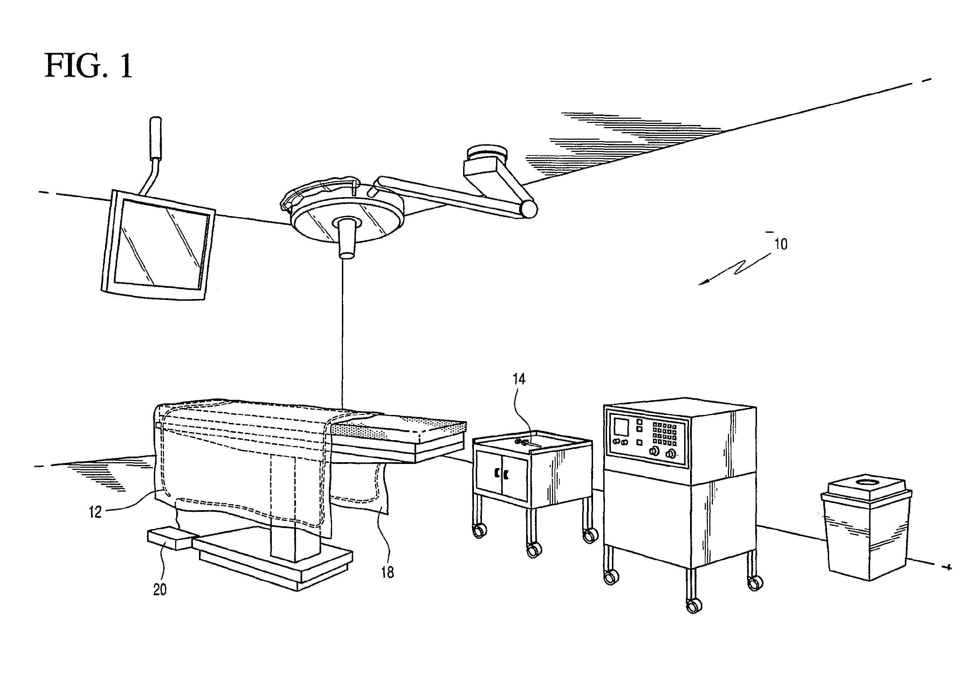

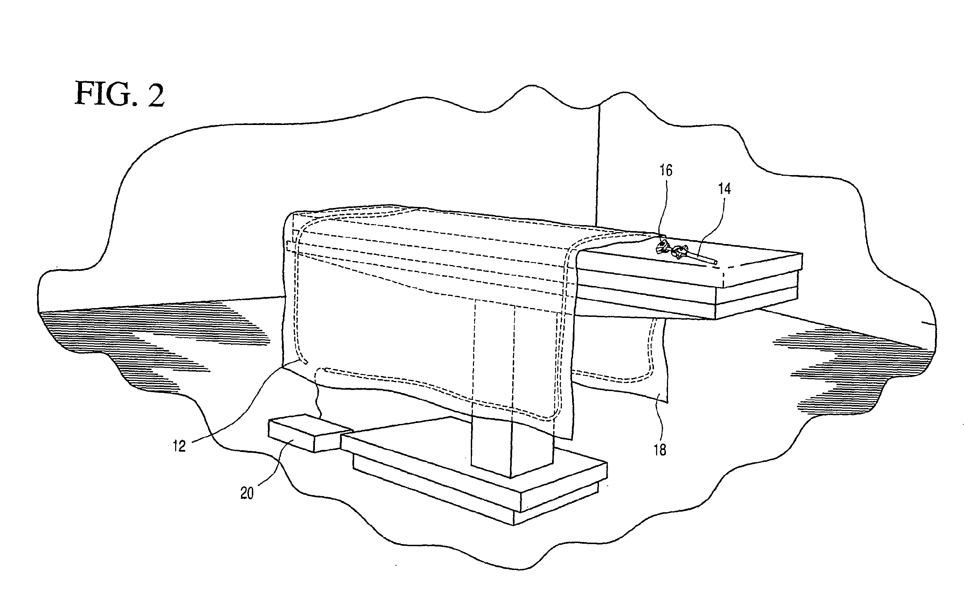

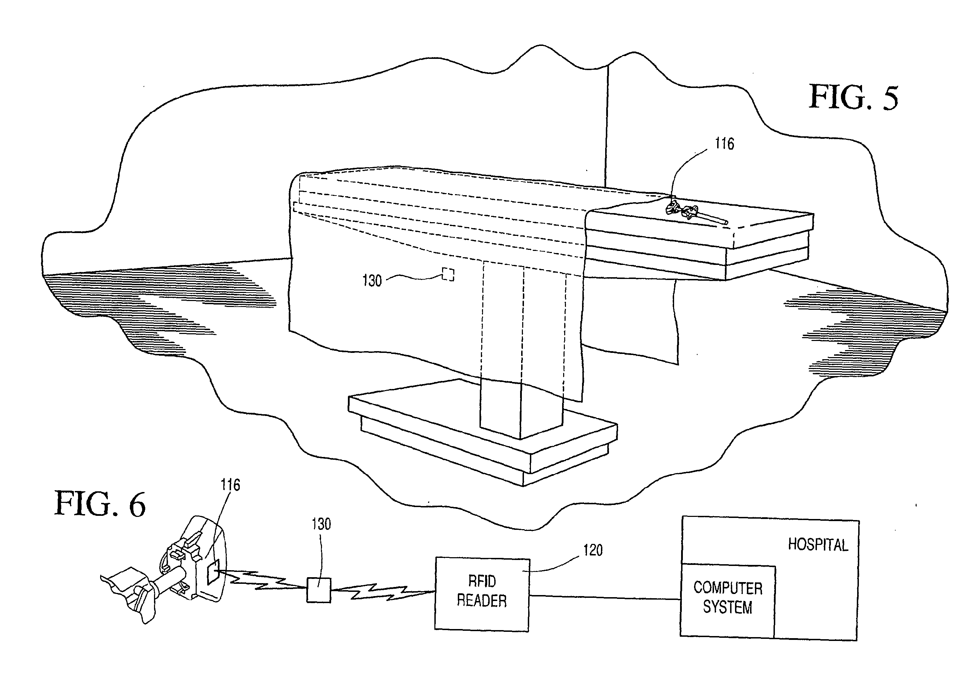

With reference to Figures 1 and 2, a first embodiment of an RFID identification system 10 in accordance with the present invention is disclosed. In accordance with this embodiment, an RFID antenna 12 is incorporated into the perimeter of the anticipated operating site, for example, of a patient drape 18, such that medical devices 14 with RFID tags 16 incorporated into or onto them can be detected when used within the sterile field defined by the antenna 12. As briefly mentioned above and as discussed below in greater detail, a sterile patent drape 18 could have a wired RFID antenna 12, or other sensor, attached or embedded into it and subsequently attached at time of placement on a patient through connecting leads to either an RFID reader 20 that then transmits to a data collection reader the information pertaining to all tagged devices that entered the draped surgical site to capture a complete history and record of devices used for billing inventory, replenishment, reconciliation/counting post procedure, etc. Although an RFID reader is disclosed for use in accordance with a preferred embodiment of the present invention, it is contemplated the RFID reader could be replaced with an active RFID tag 130 which gathers information from the passive RFID tags 116 and then forwards this information to a remotely located RFID reader 120 as shown with reference to Figures 5 and 6.

More particularly, and in accordance with a preferred embodiment of this concept as shown with reference to Figures 1, 2, 3, and 4, an RFID reader 20 with an associated RFID antenna 12 is placed in the vicinity of the operating site. More particularly, the RFID antenna 12 is oriented to surround the operating site for monitoring the passage of RFID labeled medical products 14 into or out of the operating site. For example, and as discussed above, the RFID antenna 12 is incorporated into the operating room patent drape 18. This provides a discrete mechanism for bringing the RFID identification system 10 of the present invention into the operating room and does not require a retrofitting of the existing operating rooms to incorporate such structure.

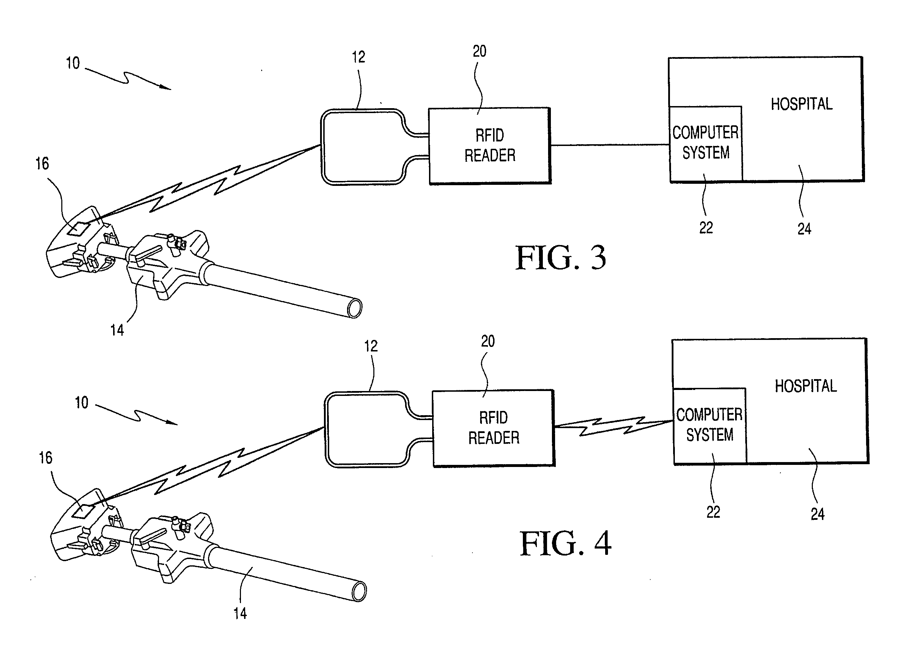

The RFID antenna 12 is linked to the RFID reader 20, which is ultimately linked to a computer system 22 of the hospital or medical facility 24 utilizing the present RFID system 10. As those skilled in the art will certainly appreciate, the RFID reader 20 is preferably hardwired to the RFID antenna 12 while the RFID reader 20 is either hardwired (see Figure 3) or wirelessly (see Figure 4) connected to the computer system 22 for the transmission of information thereto.

With the RFID antenna 12 and RFID reader 20 in place within the operating room, passage of RFID labeled medical products 14 into and out of the area encircled by the RFID antenna 12 will be identified and reported to the computer system 22 for accounting and maintenance purposes. In fact, the information transferred to the computer system 22 may be utilized for a wide variety of purposes, including, but not limited to, detecting and alerting surgical staff if a previously used medical device has left the sterile field and is now being re-used, identifying if a medical device about to be used is subject to a recent recall notice, and enabling the detection of medical devices remaining in the sterile field at the conclusion of the procedure to ensure that stray medical devices are not left behind when closing up the patient as well as ensuring actual counts entering the sterile field have in fact left it. This point of use data would also be useful to trigger an inventory replenishment from the manufacturer or distributor of that medical device which would not be desired to do until it is known that the device was actually used.

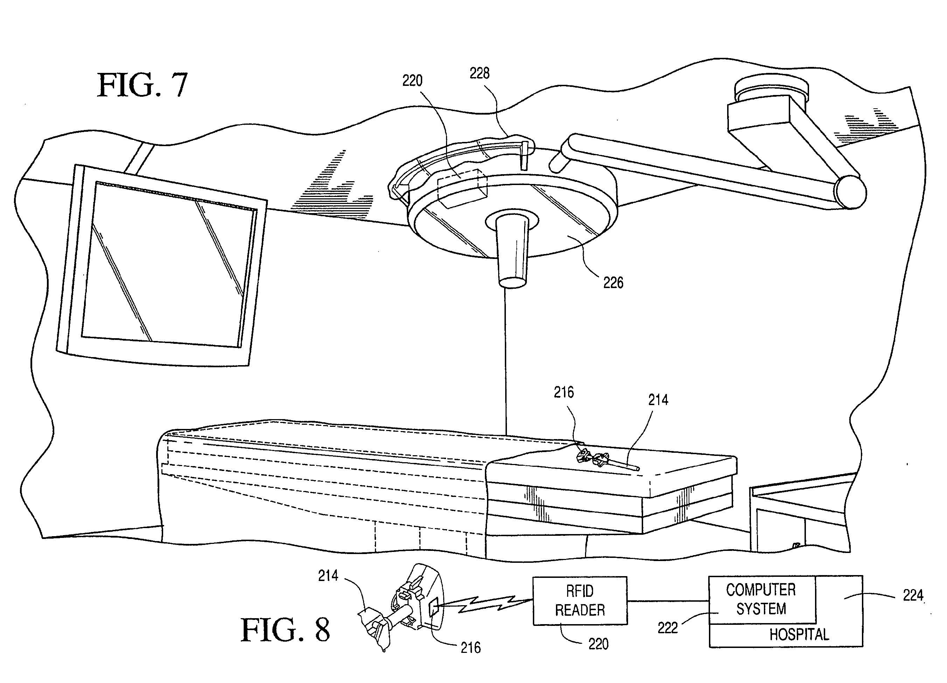

In accordance with an alternate embodiment and with reference to Figures 7 and 8, an RFID tag 216 with unique information about a medical device 214 is integrated into or onto a medical device 214 such that an RFID reader 220 is able to pick up the medical device 214 as it enters or exits the area under the monitoring of the RFID reader 220. The RFID reader 220 will be mounted above or below the procedure area.

For example, and in accordance with a preferred embodiment of the present invention, the RFID reader 220 is mounted within the surgical light 226 or disposable light handle cover 228. It is contemplated such an embodiment may be implemented by incorporating a permanent RFID reader 220 within the surgical light 226. In accordance with such an embodiment, and as discussed above with regard to the other embodiment, the RFID reader 220 would then identify the passage of an RFID labeled medical product 214 into and out of the area monitored by the RFID reader 220, and this information will be reported to the computer system 222 for accounting and maintenance purposes. As with a prior embodiment, the reporting of the identified information to the computer system 222 of the hospital or medical facility 224 may be achieved by either a wired connection or a wireless connection as is well known to those skilled in the art.

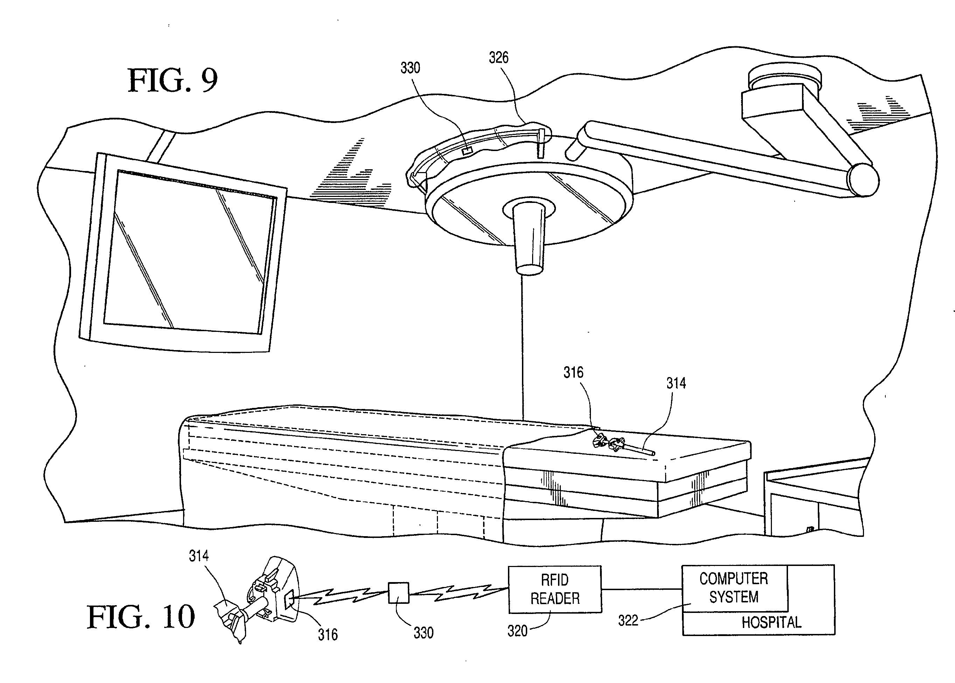

In accordance with an alternate embodiment, and with reference to Figures 9 and 10, a reusable active RFID tag 330 could be placed inside a disposable light handle cover 326 such as is typically used on the market today. The active RFID tag 330 would interrogate any passive RFID tags 316 secured to medical devices 314 entering its field of signal and relay that to a nearby RFID reader 320. The RFID reader 320 would then identify, via the transmission by the active RFID tag 330 mounted within the disposable handle cover 326, the passage of an RFID labeled medical product 314 into and out of the area monitored by the active RFID tag 330, and this information will be reported to the computer system 322 for accounting and maintenance purposes. As with a prior embodiment, the reporting of the identified information to the computer system 322 of the hospital or medical facility may be achieved by either a wired connection or a wireless connection as is well known to those skilled in the art.

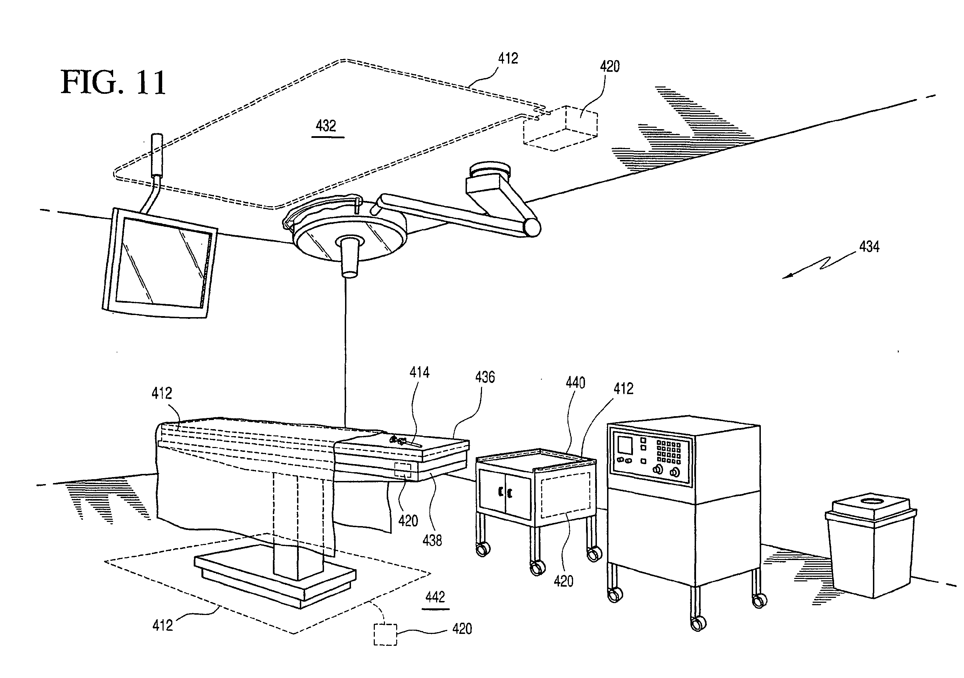

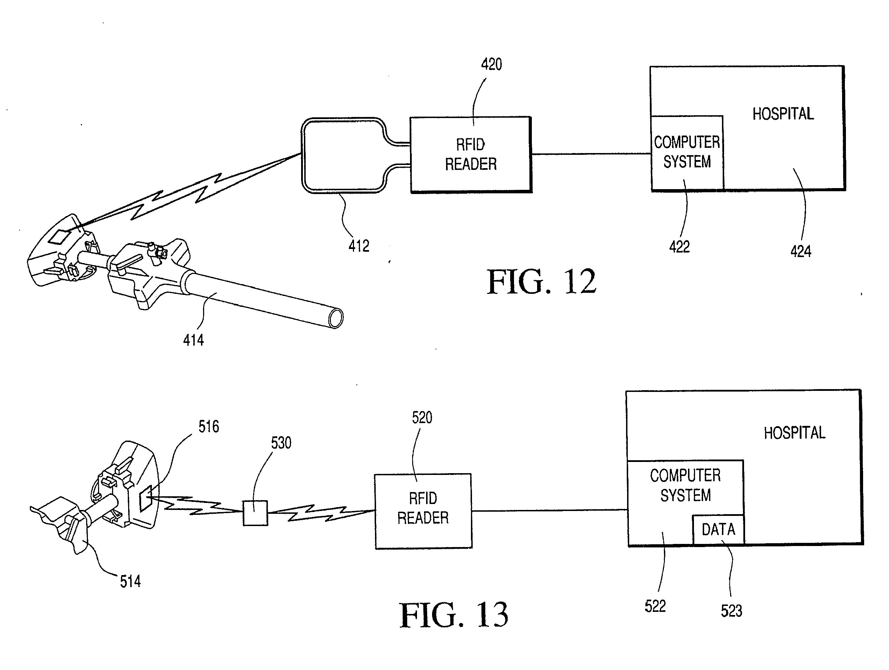

In conjunction with an alternate embodiment, and with reference to Figures 11 and 12, the RFID reader 420 may be incorporated within the ceiling 432 of the operating room 434, within the perimeter 436 of the surgical table 438, within the mayo stand 440 where medical devices 414 are placed upon removal from packaging or within (or onto) the floor area 442 around the surgical field. The area covered by the RFID reader 420 is controlled and expanded by linking the RFID reader 420 to a wired cable or wire similar to an antenna 412 which is spread to encompass the desired area within the ceiling 432 of the operating room 434, about the perimeter 436 of the surgical table 438, within the mayo stand 440 where medical devices 414 are placed upon removal from packaging, or within (or onto) the floor area 442 around the surgical field.

In accordance with such an embodiment, and as discussed above with regard to the other embodiment, the RFID reader 420, in conjunction with the RFID antenna 412, would then identify the passage of an RFID labeled medical product 414 into and out of the area monitored by the RFID reader 420, and this information will be reported to the computer system 422 for accounting and maintenance purposes. As with a prior embodiment, the reporting of the identified information to the computer system 422 of the hospital or medical facility 424 may be achieved by either a wired connection or a wireless connection as is well known to those skilled in the art.

Regardless of where the RFID reader 420 and RFID antenna 412 are positioned, the RFID sensing field as defined by the RFID reader 420 and associated RFID antenna 412 would replicate the actual sterile field. The positioning of the sensing mechanism, whether it is the active cable antenna 412 or actual RFID reader 420 is key to ensuring the actual sterile field is duplicated as closely as possible. This ensures that medical devices 414 in unopened packages passing nearby are not detected and misinterpreted as being used when in fact they may be returned unused to inventory following the procedure.

As discussed above with regard to the embodiment described with reference to Figure 13, reusable active RFID tags 530 as described above could also be used to interact, interrogate and relay passive device RFID tag 516 information to a system RFID reader 520 and database 523 of the hospital (or medical facility) computer system 522 for storage of the history data for medical devices 514 that entered the sterile field for that procedure. Through the present RFID sensing technology it is also possible to detect and alert surgical staffs if a previously used medical device has left the sterile field and is now being re-used. It is also possible to identify if a medical device about to be used is subject to a recent recall notice. It is further contemplated the present invention may enable the detection of medical devices remaining in the sterile field at the conclusion of the procedure to ensure that stray medical devices are not left behind when closing up the patient as well as ensuring actual counts entering the sterile field have in fact left it. This point of use data would also be useful to trigger an inventory replenishment from the manufacturer or distributor of that medical device which would not be desired to do until it is known that the device was actually used.

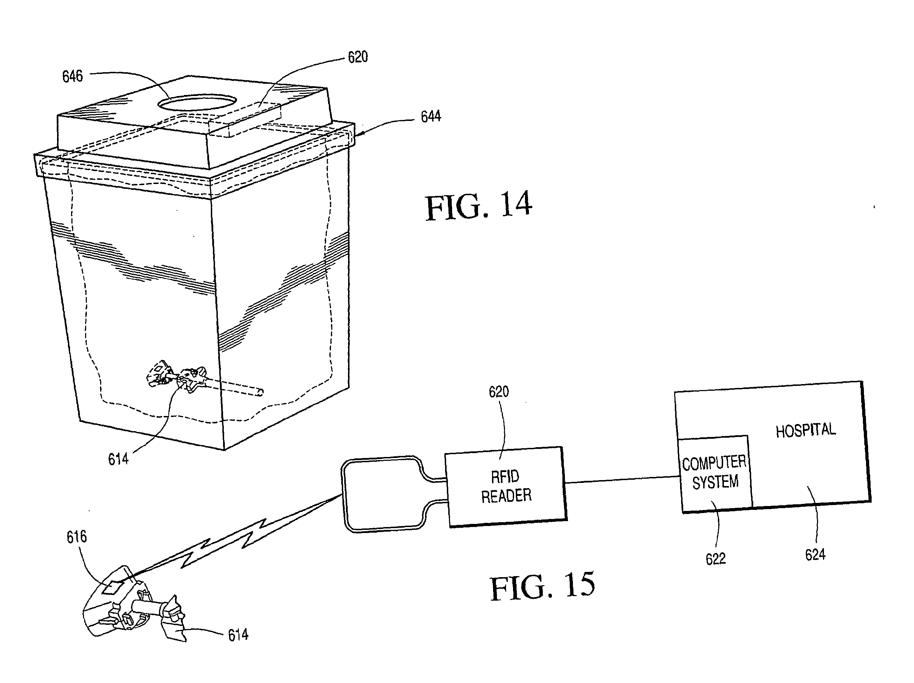

In accordance with yet another embodiment, and with reference to Figures 14 and 15, an RFID tag 616 applied to a medical device 614 may include a unique serialized identifier. A challenge in an operating room setting is capturing the point of use of a medical device. One concept for doing this is to define the point of use as the disposal of the medical device 614 into a red bag waste container 644. The waste container 644 is configured to include an RFID reader 620 either at or near the opening 646 to read and detect any medical devices 614 entering the container 644. The RFID reader 620 would then identify the passage of an RFID labeled medical product 614 into and out of the container 644 monitored by the RFID reader 620, and this information will be reported to the computer system 622 for accounting and maintenance purposes. As with a prior embodiment, the reporting of the identified information to the computer system 622 of the hospital or medical facility 624 may be achieved by either a wired connection or a wireless connection as is well known to those skilled in the art.

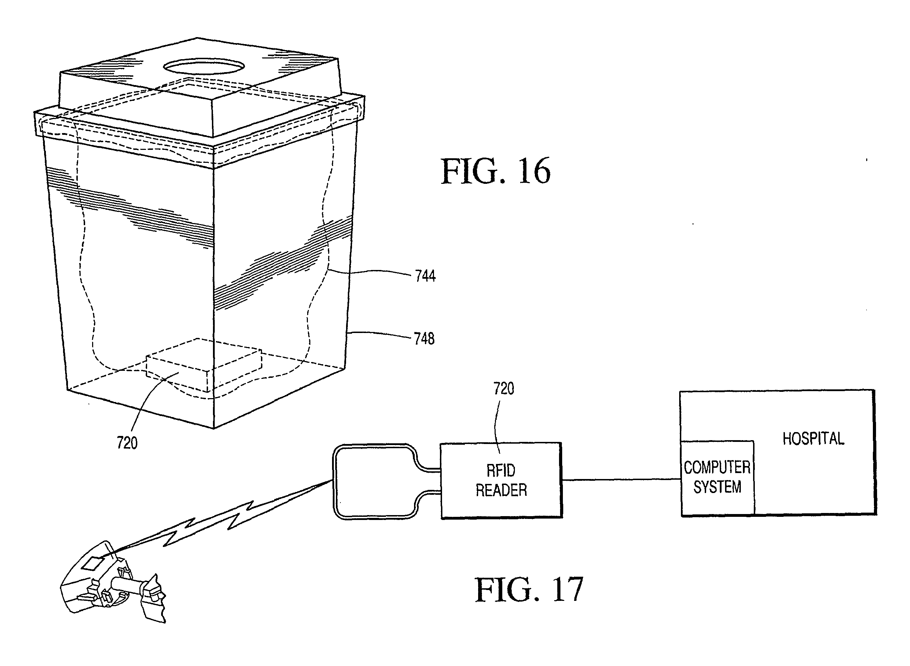

It is further contemplated in accordance with this embodiment, and with reference to Figures 16 and 17, that the RFID reader 720 may be positioned such that the entire waste bag 744 would be scanned to detect the entire contents at a given point and time. For example, the RFID reader 720 could be positioned onto the waste bag stand 748 such that a waste bag 744 is placed over it and the RFID reader 720 is capable of reading through the bag 744. This would also keep the RFID reader 720 clean by placing it under the bag 744 on the stand 748.

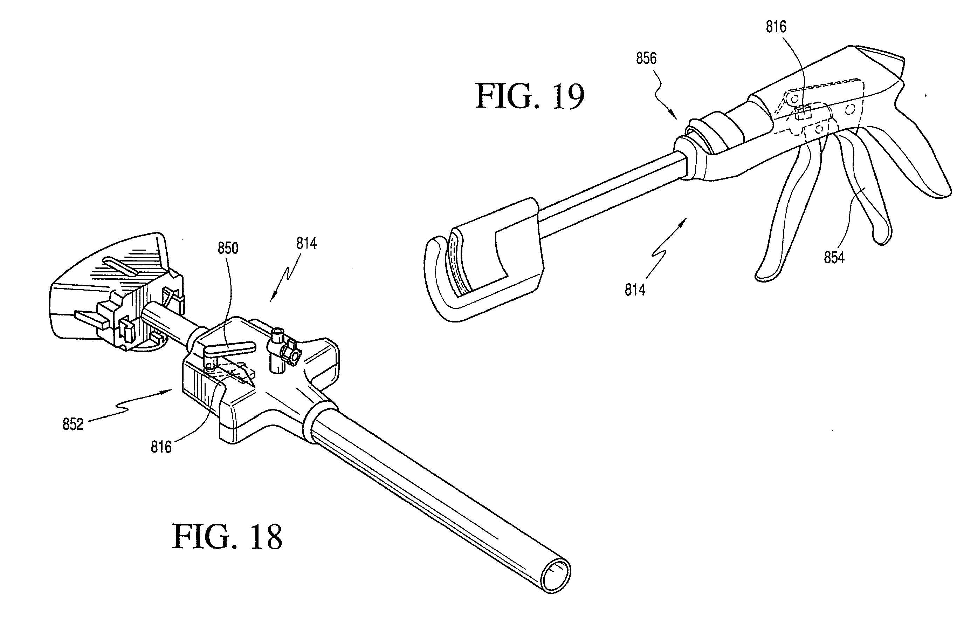

In accordance with yet a further embodiment of the present invention, and with reference to Figures 18 and 19, an RFID tag 816 is attached to the inside or outside of a single use medical device 814 for the purpose of detecting the point in time when it was first used. Many single use medical devices 814 have actuation mechanisms built into them whereby they have buttons and triggers, as well as other moving parts that activate, turn on or fire the devices. An example of such a mechanism would be an arming mechanism 850 of a trocar 852 (see Figure 18) or trigger 854 to fire an endoscopic stapling device 856 (see Figure 19).

In accordance with the present invention, the moving parts of these medical devices 814 would be utilized to interrupt or alter the circuitry of an RFID tag 816 such that the time/date of activation of the moving part 850, 854 is recorded by the chip within the RFID tag 816.

This information would then be transmitted to a local RFID reader 820. The RFID reader 820 would then identify the activation event of the RFID labeled medical product 814, and this information will be reported to the computer system 822 for accounting and maintenance purposes. As with a prior embodiment, the reporting of the identified information to the computer system 822 of the hospital or medical facility 824 may be achieved by either a wired connection or a wireless connection as is well known to those skilled in the art.

As those skilled in the art will certainly appreciate, RFID tags 816 consist of an electronic chip 858 connected to an antenna 860 which is typically either a metallic inlay wire or more recently, printed with conductive inks. In accordance with a preferred embodiment of the present invention, the moving parts 850, 854 within the medical instrument 814 are designed to move across specific points of the antenna 860 and/or secondary printed circuit 860 of the RFID tag 816 to either disrupt or momentarily connect two circuits or permanently destroy through abrasion or other means for the antenna or circuit to function. When such an event occurs, the date and time of the occurrence is recorded onto the electronic chip 858 for future interrogation and decoding by an RFID reader 820 and associated computer system 822 as part of any quality investigation around the historical use of the particular medical device. Since it is known that the reuse of single use medical devices can increase their failure rate, the recording of this documented use of medical instruments can assist in protecting patients, hospitals, medical facilities and manufacturing companies from injuries and/or liability in instances where devices are reprocessed by third parties and utilized in an authorized and dangerous manner.

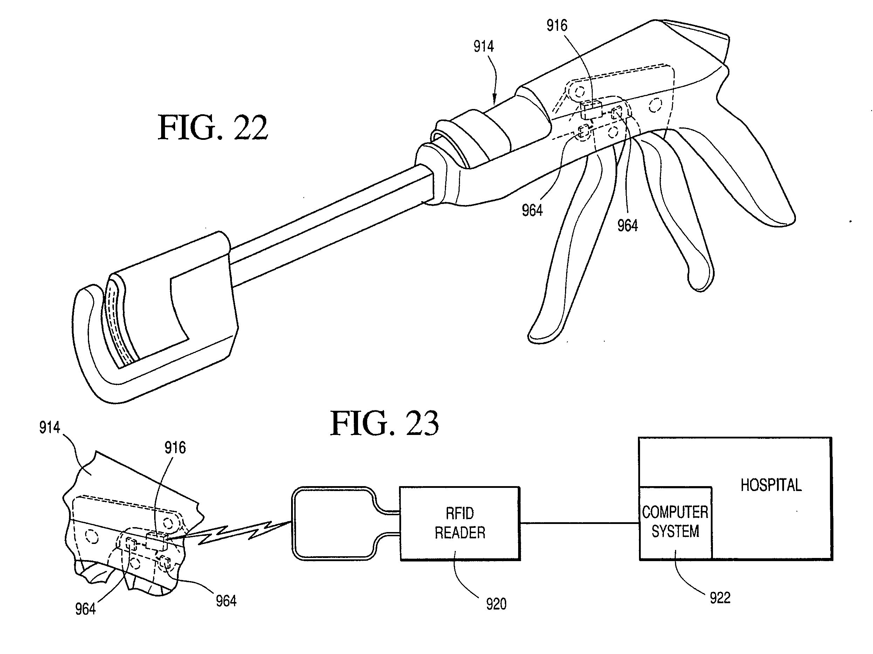

It is further contemplated with reference to Figures 22 and 23, the concepts of the present invention may be implemented such that sensors 964 are attached to the RFID tags 916 to also record specific conditions of use or misuse such as temperature, humidity, shock, vibration, GPS location, etc. Such information is also important for use in determining the history of a particular device 914. This information can further be used to trigger an electronically sent message to an RFID reader 920 and associated computer system 922 for informing users that the particular medical device 914 has experienced conditions outside those allowed by its manufacturer and should not be used.

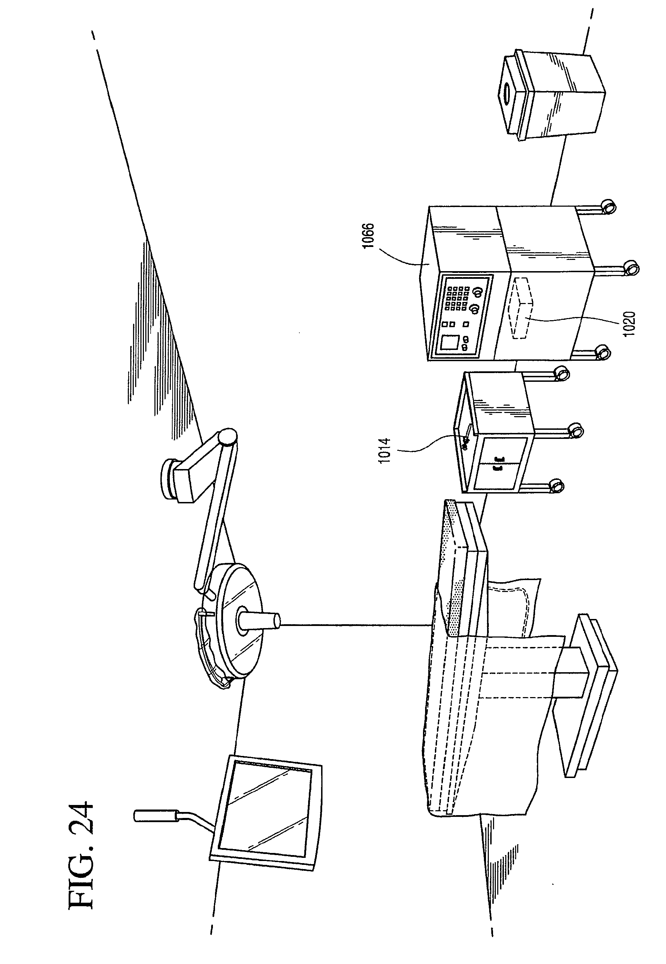

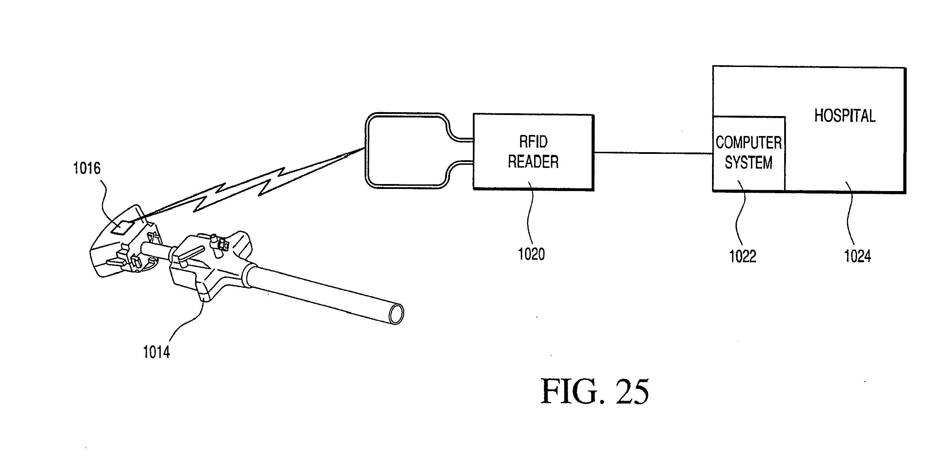

In accordance with yet a further embodiment of the present invention, and with reference to Figures 24 and 25, an RFID tag 1016 is used on or in a disposable medical device 1014 designed for one-time (or limited) use in conjunction with electronic capital equipment 1066. The electronic capital equipment 1066 is provided with an RFID reader 1020. The RFID tag 1016 is detected by the built in RFID reader 1020 within the electronic capital equipment 1066, such as, an ultrasonic harmonic generator, and the unique serial number of the RFID tag 1016 recorded electronically by the equipment 1014. The RFID reader 1020 then identifies the use of the disposable medical equipment 1014, and this information is reported to the computer system 1022 for accounting and maintenance purposes. As with a prior embodiment, the reporting of the identified information to the computer system 1022 of the hospital or medical facility 1024 may be achieved by either a wired connection or a wireless connection as is well know to those skilled in the art.

Such an implementation would establish "the initial use record" by that piece of disposable medical equipment 1014 for that specific device. Any subsequent return of the disposable medical equipment 1014 to the same facility 1024 would be detected and prevented from functioning with the electronic equipment 1066 if so desired. This could further prevent use between other pieces of electronic equipment provided they are connected to an Internet access point, or other global or local communication network, and use is reported to a host computer at the original manufacturer or retained by a third party contracted to serve on its behalf. Therefore, the subsequent use of the disposable medical equipment for a second time at an entirely different facility could still be detected and blocked through the equipment's access to records and data of device use worldwide.

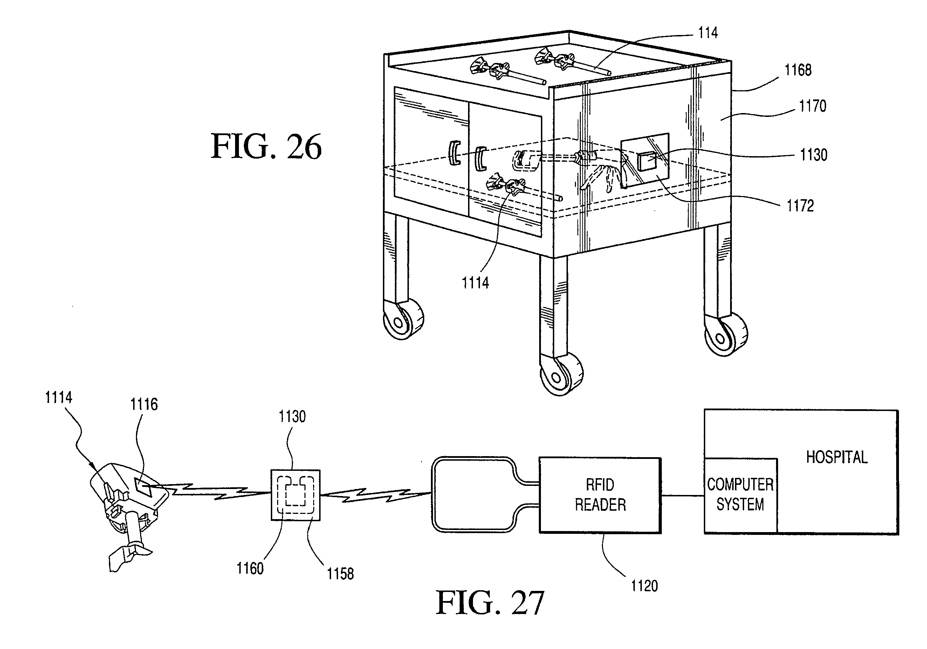

In accordance with yet a further embodiment, and with reference to Figures 26 and 27, RFID tags 1130 are incorporated into a hospital case cart 1168 for delivery of surgical devices 1114 to an operating room. The RFID tag 1130 consists of an electronic chip 1158 connected to an antenna 1160, both of which are attached to medical devices 1114 for reading by an external reader 1120. RFID tags 1130 may be self powered with batteries to periodically sent their signal and/or data to a reader or wireless network.

It is known that currently numerous hospitals utilize case carts 1168 for the delivery of surgical supplies/devices 1114 to an operating room for a scheduled procedure. These case carts 1168 are typically metal which can cause problems for reading RFID tags 1116 on items 1114 within the interior of the cart 1168 through external RFID readers 1120.

As such the present concept applies an active battery powered RFID tag 1130 to the external surface 1170 of the cart 1168 over an open window 1172 of the cart 1168 (or over a material covered window that is transparent to radio waves such as thin plastic, etc.), such that tagged items 1114 inside the cart 1168 can be detected by the external mounted active RFID tag 1130.

The internally tagged items 1114 would be RFID tagged 1116 with lower cost passive tags requiring an external power provided to send their identification and other product data to a requesting device. The active RFID tag 1130 on the cart 1168 would provide this power to activate the internal passive RFID tags 1116 and interrogate them to send their identification data to the active RFID tag 1130. The active RFID tag 1130 would then relay both its own unique identifying number as well as its contents numbers upon request to an RFID reader 1120.

While one can simply track the cart 1168, emergency situations occur wherein personnel may have an urgent need to locate a specific item that was originally not planned for that procedure or accidentally dropped rendering it non-sterile or previously removed from a cart by another worker, etc. whereby having complete and accurate data readily available at all times becomes vital. Likewise it is becoming a practice that hospitals have third parties prepare case carts 1168 packing them with a planned list of medical devices required for a certain procedure and deliver it to an operating room at a specified scheduled time. The location of the cart can be tracked over a wireless network within the hospital as the active RFID tag 1130 on the cart 1168 will transpond its identification data at preprogrammed tine intervals or as the RFID tag 1130 is interrogated by the network as it is moved through the facility or designated locations (such as the receiving dock, operating room, corridor, central supply or stock room/warehouse).

Through the use of the described RFID tagging methodology, the hospital can readily verify the accuracy of the contents electronically prior to or during setup for the procedure before the patient or surgeon arrive in the room. Following the conclusion of the procedure, it is then likewise readily electronically recordable very easily which items remain in the case cart for delivery back to inventory. The inventory location then verifies simply by scanning the case cart tag which contents are inside to provide ongoing security verifications to prevent theft. Likewise the active RFID tag 1130 can connect to and record the time events and other data associated with the opening of the case cart 1168 doors and if so programmed can detect and record the personnel doing so provided they are tagged and associated with RFID employee badges. Use of the present embodiment can dramatically reduce the time and effort required. The present procedure can also increase the accuracy of preparing surgical supplies for surgical procedures. It also greatly reduces the currently increasing risk of security and theft that can occur within a healthcare facility.

While the preferred embodiments have been shown and described, it will be understood that there is no intent to limit the invention by such disclosure, but rather, is intended to cover all modifications and alternate constructions falling within the spirit and scope of the invention.

## Claims

1. An RFID identification system, comprising:
an RFID tag associated with a medical device;
an RFID attenna for receiving signals issued by the RFID tag;
an RFID reader identifying an RFID labeled medical device; and
a computer system in communication with the RFID reader.

2. The RFID identification system according to claim 1, wherein the RFID antenna is incorporated into a perimeter of an anticipated operating site.

3. The RFID identification system according to claim 2, wherein the RFID attenna is incorporated into a patient drape.

4. The RFID identification system according to claim 1, wherein the RFID reader is hardwired to the computer system.

5. The RFID identification system according to claim 1, wherein the RFID reader is wirelessly connected to the computer system.

6. The RFID identification system according to claim 1, wherein the RFID tag is provided with unique information about the medical device such that an RFID reader is able to pick up the medical device as it enters or exits the area under the monitoring of the RFID reader.

7. The RFID identification system according to claim 6, wherein the RFID reader is mounted within a surgical light.

8. The RFID identification system according to claim 6, wherein the RFID reader is mounted within a disposable light handle cover.

9. The RFID identification system according to claim 1, wherein the RFID reader is incorporated within the ceiling of the operating room.

10. The RFID identification system according to claim 1, wherein the RFID reader is incorporated within the perimeter of the surgical table.

11. The RFID identification system according to claim 1, wherein the RFID reader is incorporated within the mayo stand where medical devices are placed upon removal from packaging.

12. The RFID identification system according to claim 1, wherein the RFID reader is incorporated within or onto the floor area around the surgical field.

13. The RFID identification system according to claim 1, wherein the RFID tag applied to a medical device includes a unique serialized identifier.

14. The RFID identification system according to claim 1, wherein the RFID reader is included with the waste container.

15. The RFID identification system according to claim 1, wherein the RFID tag is attached to the inside or outside of a single use medical device for the purpose of detecting the point in time when it was first used.
